# EUROPEAN PATENT SPECIFICATION

(11) **EP 0 737 178 B1**
(45) Date of publication and mention of the grant of the patent: **25.03.1998**
(21) Application number: 95908425.2
(22) Date of filing: 30.12.1994
(51) Int. Cl.: C07C 13/43

(54) **RECOVERY OF REACTANTS AND PRODUCTS IN PRODUCTION OF ALKENYL BRIDGED RING COMPOUNDS**
ZURÜCKGEWINNUNG VON REAKTANTEN UND PRODUKTEN BEI DER HERSTELLUNG VON RINGVERBINDUNGEN MIT ALKENYLBRÜCKE
RECUPERATION DE REACTIFS ET DE PRODUITS POUR PRODUIRE DES COMPOSES CYCLIQUES A PONTAGE ALCENYLE

(30) Priority: 30.12.1993 US 175443
(43) Date of publication of application: 16.10.1996
(73) Proprietor: EXXON CHEMICAL PATENTS INC., Baytown, TX 77520-5200 (US)
(72) Inventor: LATTNER, James, Richardson, Seabrook, TX 77586 (US); SANCHEZ, Leonel, Eduardo, Valles de Vista Hermosa Guatemala (GT); BECKER, Christopher, Lynn, Baton Rouge, LA 70806 (US); DEVOY, Bruce, Charles, Baton Rouge, LA 70808 (US)
(74) Representative: Bawden, Peter Charles
(86) International application number: PCT/US94/14997
(87) International publication number: WO 95/18087

(56) References cited:
- FR-A- 2 242 351
- US-A- 3 590 089
- US-A- 3 728 406

## Description

The present invention relates to an improved process for recovering alkenyl bridged ring compounds produced by reacting olefins with cyclic diolefins and for recovering and using the unreacted cyclic diolefins to produce additional amounts of the alkenyl bridged ring compounds. More particularly, but not exclusively, the present invention relates to an improved process for recovering 5-vinyl-2-norbornene produced by reacting butadiene with 1,3-cyclopentadiene and/or dicyclopentadiene, and for recovering and recycling the unreacted 1,3-cyclopentadiene to produce additional amounts of the 5-vinyl-2-norbornene.

One way to produce an alkenyl bridged ring compound is to use a Diels-Alder reaction, which is the additive reaction of an olefin with a cyclic diolefin. For example, 5-vinyl-2-norbornene (an alkenyl bridged ring compound) may be produced by the Diels-Alder reaction of 1,3 butadiene (an olefin) with 1,3-cyclopentadiene (a cyclic diolefin). 5-vinyl-2-norbornene (VNB) is very desirable as it is an intermediate in the production of 5-ethylidene-2-norbornene (ENB), a termonomer used in the production of ethylene-propylene-diene monomer (EPDM) rubbers.

The conditions necessary to bring about the Diels-Alder reaction of 1,3-butadiene (BD) with 1,3-cyclopentadiene (CPD) are well known in the art. In particular, BD may be contacted with CPD in the liquid phase at temperatures of from 100° to 200°C and a pressure of from 1.0-2.0 MPa (150 to 300 psi). It is not necessary to use a catalyst to advance the Diels-Alder reaction. The reaction is generally completed in 0.1 to 100 hours and is usually conducted under an inert atmosphere. Preferably, the reaction may be conducted in a liquid state, most preferably in a liquid-full reaction vessel.

When making VNB the preferred reaction occurs between BD and CPD, however, undesirable polymerization reactions may also occur. For example, BD monomers may react with each other to form polymers. Similar polymerization reactions may occur between CPD monomers. Undesirable (heavy) by-products include dimerization of BD to form 4-vinyl-1-cyclohexene (VCH), rearrangement of VNB to form 4, 7, 8, 9-tetrahydroindene (THI) and Diels-Alder adducts of BD or CPD with VNB, VCH, or THI (hereinafter referred to as "trimers of BD/CPD").

CPD will readily react with itself to form dicyclopentadiene (DCPD). In turn, DCPD will crack upon heating, back to the CPD monomer. Therefore, both CPD and/or DCPD can be used as the cyclic diolefin feedstock in the production of alkenyl bridged ring compounds. As used herein, the term "(di)cyclopentadiene" refers to cyclopentadiene, dicyclopentadiene or mixtures thereof in the reaction mixture. In the present application, cyclopentadiene and dicyclopentadiene are regarded as the same substance in the calculation of the conversion of raw materials and, hence, the transformation of cyclopentadiene into dicyclopentadiene, and vice versa is not considered a conversion.

Certain compounds are known which suppress or inhibit the undesired polymerization reactions. Any one or combination of these inhibitor compounds may be added to the reactants in order to produce more VNB from the same amount of starting material and to avoid plugging certain parts of the reaction apparatus with the high molecular weight polymers which might otherwise be formed. Many inhibitor compounds are known in the art including 2,6-di-t-butyl-p-cresol, diphenylnitrosamine, and N-substituted p-phenylenediamines.

In addition to the problems caused by fouling, the undesired by-products are difficult to separate from VNB which create additional problems in the production process of making VNB.

As taught by US-A-3,728,406, improved selectivity during the production of VNB, or any alkenyl bridged ring compounds, can be achieved by two methods. First, the starting material may be limited to the CPD monomer, rather than the DCPD dimer. This results in higher conversion of the (di)cyclopentadiene to VNB. Second, the reaction can be discontinued early after only a relatively small portion of the total CPD and/or DCPD has been consumed. One of the disadvantages of this process is that a substantial amount of residual DCPD is left in the reactor effluent. However, this residual DCPD can be recovered to produce additional alkenyl bridged ring compound. Steps to recover the DCPD include: (1) vacuum distillation to separate the DCPD from the THI and trimers of BD/CPD, or (2) selectively cracking the DCPD to CPD, which is then easily separated from the THI and trimers of BD/CPD because it is much more volatile. Unfortunately, either method requires additional processing equipment and the conditions used to crack DCPD typically result in fouling of the equipment.

FR-A-2242351 deals with the preparation of 2-(1-propenyl)-norbornene-5 by reaction of cyclopentadiene and piperylene; the process also comprises the separation by distillation of the reactor effluent. The final bottom stream comprises dicyclopentadiene, which can be recycled to the reaction step, possibly after thermal cracking.

It would be advantageous if a simple and economical method could be devised to recover the residual dimer of the cyclic diolefin from the production of an alkenyl bridged ring compound, by cracking the dimer to cyclic diolefin monomer, and recycle the monomer to produce more of the desired alkenyl bridged ring compound without fouling of the equipment.

### SUMMARY OF THE INVENTION

The present invention relates to a continuous process which utilizes a distillation column to recover the reactants and products from the Diels-Alder reaction of a cyclic diolefin and an olefin to produce an alkenyl bridged ring compound. The first embodiment includes the single process step of a distillation column, to which the reaction effluent is fed. The unreacted cyclic diolefin and olefin are removed from the column as overhead products and preferably are recycled to the reaction feed mixture to produce additional alkenyl bridged ring compound. The alkenyl bridged ring compound is removed as a side stream at a location below the feed to the column, which can then be purified further by conventional means. The higher boiling reaction by-products and dimer of the cyclic diolefin are treated in the bottom of the column, such that the dimer of the cyclic diolefin is selectively "cracked", or converted back to the monomer, which then travels to the top of the column and is recovered with the optional recycle stream to the reaction feed mixture.

The second embodiment includes an additional process step whereby the reaction effluent is first flashed in a pre-flash column to perform a crude separation prior to being fed to the distillation column. The Diels-Alder reaction effluent is fed to the flash column, which separates the cyclic diolefin dimer from light boiling reactants and/or diluent, and feeds the recycle distillation in two places. The overhead from the flash column which is depleted in dimeric cyclic diolefin is fed to the recycle column at a location above the sidestream draw, and the bottoms from the flash column which is enriched in dimeric cyclic diolefin is fed at a location below the sidestream draw. This allows the desired alkenyl bridged ring compound to exit the recycle column sidestream without containing any of the unreacted cyclic diolefin dimer and eliminates the need for further purification as is required for the first embodiment.

A feature of this invention is that the desired alkenyl bridged ring compound is separated from both the monomer and dimer of the unconverted cyclic diolefin feed, while at the same time the dimer of the cyclic diolefin is separated from the heavy reaction by-products by conversion to the cyclic diolefin monomer. The cyclic diolefin monomer from both the reaction effluent, as well as that converted from the dimer, are simultaneously recovered in the present invention, preferably for recycle to the reaction feed mixture. Additional alkenyl bridged ring compound can then be produced from this recovered cyclic diolefin monomer.

In the second embodiment, the primary advantage obtained by the operation is that nearly all of the dimer is recovered in the bottoms of the recycle distillation column, which is subjected to conditions which crack the dimer back to the monomer, which travels up the column and exits the recycle column with the overhead product stream for return to the Diels-Alder reactor.

In either embodiment, the cyclic diolefin reactant preferably comprises monomeric and dimeric cyclic diolefin. Also, at least some of the make up of cyclic diolefin reactant is provided by feeding dimeric cyclic diolefin to the distillation column, cracking the dimer to monomer in the column bottoms and recovering the make up monomeric cyclic diolefin as column overhead.

In either embodiment, the additional or make up monomeric cyclic diolefin is recovered without removing the bottoms from the distillation column. Also, the additional or make up cyclic diolefin is recovered as distillation column overhead.

In either embodiment, the bottoms is preferably cracked for at least fifteen minutes, and more preferably for at least one hour.

In both embodiments, the bottoms are preferably cracked at a pressure of from 3.5-207 kPa (5-30 psia), and more preferably at a pressure of from 75.8 - 117.2 kPa (11-17 psia). Also, the bottoms are preferably cracked at a temperature of about 170-190°C (338-374°F).

In either embodiment, the cyclic diolefin preferably comprises 1-methyl-1,3-cyclopentadiene, 2-methyl-1,3-cyclopentadiene, 5-methyl-1,3-cyclopentadiene, 1,3-cyclopentadiene, dicyclopentadiene or mixtures thereof.

In either embodiment, the most preferred reactants and product comprise the cyclic diolefin as 1,3-cyclopentadiene, the olefin as butadiene, and the alkenyl bridged ring compound as 5-vinyl-2-norbornene.

### BRIEF DESCRIPTION OF THE DRAWINGS

**Fig. 1** is a schematic diagram which illustrates the equipment and the flow of a process according to the first embodiment of the present invention.

**Fig. 2** is a schematic diagram which illustrates the equipment and the flow of a process according to the second embodiment of the present invention.

**Fig. 3** is a graph of heat transfer coefficient of the heat exchanger used in Example I.

### DETAILED DESCRIPTION OF THE INVENTION

Although, for convenience of discussion, the foregoing and following description often refers to the separation of reactants and products from the production of VNB, the invention is not limited to such use. Many modifications may be made to the embodiments described which are illustrative only. The present invention may be used for producing any alkenyl bridged ring compound made from a Diels-Alder reaction of a cyclic diolefin, such as CPD and/or DCPD or methyl cyclopentadiene and/or dimethyl-cyclopentadiene, with an olefin, such as ethylene, acetylene, propylene, 1,3-butadiene, 1,2-butadiene, piperylene, or isoprene.

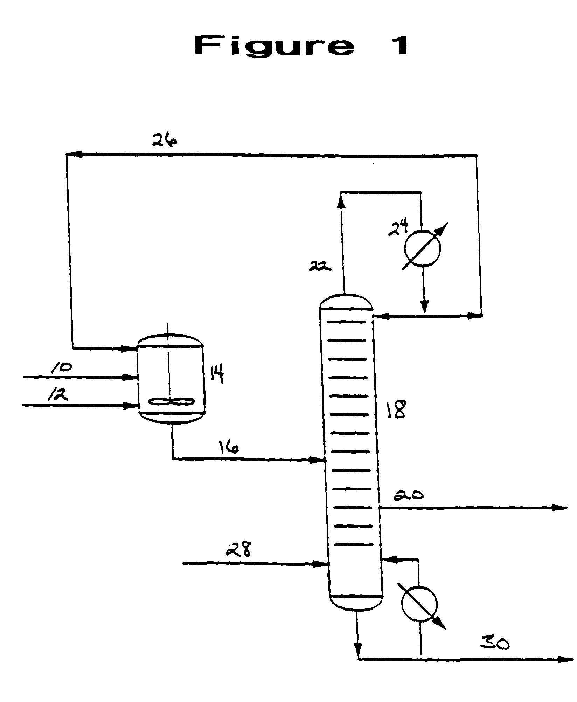

Referring now to **Fig. 1**, as the first embodiment of the present invention, this is discussed, for convenience, in terms of VNB production. However, the arrangement and operating conditions are equally applicable to all generic and specific embodiments of the invention and are intended to be continued as having such applicability to a more general description of the invention. Thus, in order to produce VNB, BD and (di)cyclopentadiene are fed via lines **10** and **12**, respectively, into a Diels-Alder reactor **14** and reacted using known methods. Preferably the reaction is stopped when about 50 percent of the (di)cyclopentadiene feed has been converted, as described in US-A-3,728,406. As noted in US-A-3,728,406, the reaction time required to reach the desired degree of conversion of 50 percent or lower of the (di)cyclopentadiene will vary according to reaction conditions, such as temperature, pressure, ratio of reactants, presence and amount of catalyst, if any. Thus, no absolute reaction times can be set forth. However, one skilled in the art easily should be able to determine the necessary reaction time by performing an orienting experiment using a given set of reaction conditions. Generally, reaction times are preferred to be less than one hour, but greater than ten minutes.

Once the reaction has reached the desired conversion level, the effluent from the Diels-Alder reactor may be fed via line **16** to a distillation column **18**. The effluent with typically contain BD, CPD, diluent (typically a C6-C7 aliphatic or aromatic solvent), VCH, VNB, THI, DCPD, and trimers of BD and CPD.

The distillation column **18** is preferably maintained at an operating pressure of between 34.4-206.8 kPa (5-30 psia), more preferably between about 75.8-117.2 kPa (11-17 psia). Higher pressures may result in unacceptable levels of conversion of VNB to THI and an increased potential to foul the column reboiler. Lower pressures may result in column temperatures which would be insufficient to achieve significant cracking of DCPD to CPD. The temperature in the bottom of the column is preferably maintained at least 130°C (266°F), more preferably between 170-190°C (338-374°F), and most preferably about 175°C (347°F).

The crude VNB, which typically also includes some VCH, THI, and DCPD, is removed from the column, preferably in a side stream via line **20**, which should be located at a point below the effluent feed to the column. The removal of VNB as a side stream, rather than leaving the VNB in the bottoms, reduces the undesired conversion of VNB to THI. The VNB side stream may subsequently be treated with additional distillation steps (not shown), using known procedures, to remove the lighter boiling VCH and the heavier-boiling THI and DCPD.

Under the foregoing column conditions, the lighter boiling BD and CPD from the effluent travels up the distillation column, along with the light boiling diluent, and may be removed as overhead via line **22**. In the preferred embodiment shown, the overhead is recycled to the Diels-Alder reactor **14**, e.g., via a heat exchanger **24** and recycling line **26**.

The remaining bottoms typically contains primarily THI, DCPD, and trimers of BD/CPD and other oligimers of CPD, BD, and acyclics. The bottoms may be left in the distillation column, or otherwise exposed to the foregoing column conditions, preferably for at least fifteen minutes, more preferably at least one hour. During this time an amount, preferably at least 80%, more preferably 90% or above, of the DCPD in the bottoms will crack to monomeric CPD. The resulting CPD will travel up the column and may be removed as overhead via line **22** and optionally recycled to produce additional VNB.

The resulting DCPD-depleted heavy product may be removed as bottoms via line **30**.

One surprising result when the bottoms is left in the column for a time sufficient to crack the residual DCPD, which is preferably at least fifteen minutes, is the very low polymer formation or fouling of the equipment. The literature teaches that, when trying to crack DCPD to CPD in the liquid phase, it is essential to use a high-boiling inert solvent to minimize the formation of CPD oligomers which foul the equipment. (*See* US-A-3,590,089). No such solvent needs to be added to the bottoms during cracking in the process according to the present invention. Therefore, without limiting the invention to any particular theory, it appears that the THI and trimers of BD/CPD in the bottoms behave as diluents in the cracking reaction. This result is unexpected since these trimers are not inert to additional thermal Diels-Alder reactions. Nevertheless, analysis of the reboiler heat transfer coefficient during extended runs shows that the coefficient holds fairly stable. Visual inspection of the reboiler following the total period of the run showed only a very thin film of polymer on the heat transfer surfaces. No polymer collected on the filters in the bottoms product effluent line.

Another surprising result when the bottoms is cracked is the very high DCPD cracking level achieved (approximately 90% or above) in the boiling liquid of the column bottoms when operated at atmospheric pressure and at a temperature as low as 175°C (347°F). Again, without limiting the present invention to any particular theory, it appears that the presence of high-boiling trimers of BD/CPD in the bottoms is serves to achieve the desired cracking levels without the need for external addition of a high boiling solvent.

The "in column" cracking of DCPD in the present invention can result in two additional benefits. First, the fresh make-up DCPD may be passed through the distillation column **18**, e.g., via line **28**, before being fed to the Diels-Alder reactor. This allows virtually all of the (di)cyclopentadiene feed to the Diels-Alder reactor to be monomeric CPD, rather than a mixture of CPD and DCPD. Second, when further purification of the crude VNB takes place, the portion of the VNB side stream containing DCPD can be returned to the bottoms in the distillation column **18** for cracking in the process according to the present invention.

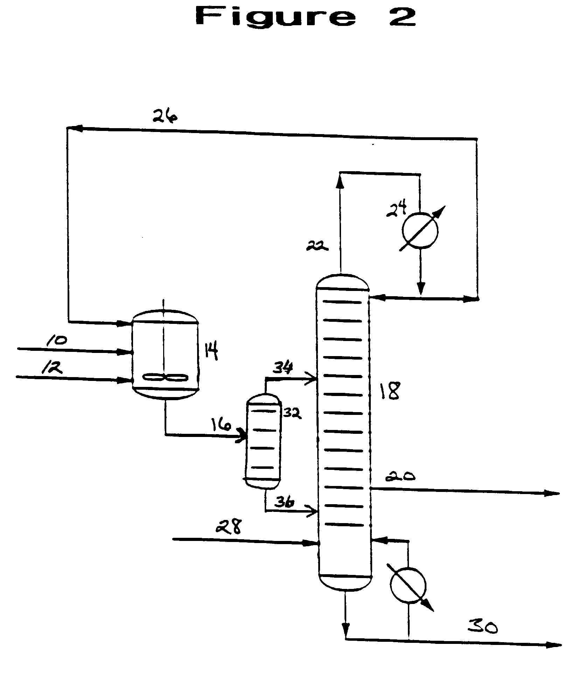

Referring now to **Fig. 2**, the second embodiment of the invention includes an improvement that substantially reduces the loss of other materials with the VNB sidestream. This is done by performing a crude separation of the Diels-Alder reaction effluent **16** prior to feeding it to the recycle distillation column **18**. A small column **32**, referred to as a "flash column" is used to separate the light-boiling diluent from the dimer. This is a very easy separation because there is a wide difference in boiling points (in the case of toluene as a diluent and DCPD as the dimer, the boiling point difference is about 60°C). The overhead stream from the flash column **34**, which is substantially free of the dimer, is fed to the distillation column **18** at a location above the sidestream draw. The bottoms stream from the flash column **36**, which is substantially free of the diluent (and lighter boiling materials), is fed at a location below the sidestream draw **20**. The Diels-Alder reaction product, such as VNB, boils between the diluent and the dimer, and will exit both the top and bottom of the flash column, which is not a problem. The recycle distillation column can now produce a Diels-Alder sidestream product that contains all of the desired Diels-Alder reaction product (such as VNB), but is free of both the dimer and the diluent, substantially reducing the losses of either material that would be unavoidable if practiced without the benefit of the flash column.

The second embodiment is preferred over the first embodiment of the present invention in that with the first embodiment, some of the dimer entering the column with the feed exits the VNB sidestream product draw, without being subjected to the desirable cracking conditions in the column bottoms. This is unavoidable, since the column feed is located above the sidestream draw, which causes some of the dimer to exit with the sidestream on its way to the bottom of the column. If the sidestream product were to be withdrawn at a location above the feed point, it could be free of the dimer, but there would be an unavoidable loss of some diluent, diene, and dienophile out with the sidestream.

In the second embodiment, the flash column may be operated in a conventional manner, using a reboiler and a condenser. Rather than using a conventional reboiler as a heat source for the flash column, some of the bottoms from the recycle column may be fed to the bottoms of the flash column, providing heat for vaporization of the column liquid by direct contact heat exchange. In addition, the condenser may be eliminated by utilizing a small portion of the liquid distillate from the recycle column as reflux for the flash column.

The present invention is further described in the following examples.

### EXAMPLE I

The process of the invention was demonstrated as described below in a continuous pilot unit for 6 weeks.

5.9 kg/hr (13.0 lb/hr) BD and 6.0 kg/hr (13.2 lb/hr) (di)cyclopentadiene (mostly CPD) and 6.4 kg/hr (14.1 lb/hr) of toluene (a diluent) were fed into a Diels-Alder reactor and reacted in order to produce VNB. The reactor was operated at a temperature of 154°C (310 °F) and at a pressure of 2,067 kPa (300 psi) to keep all reactants in the liquid phase. The residence time of the reactor was one hour, which resulted in a 33% conversion of the (di)cyclopentadiene.

The effluent from the Diels-Alder reactor was fed to a distillation column. The effluent contained BD, CPD, toluene, VCH, VNB, THI, DCPD, and trimers of BD/CPD.

The distillation column was maintained at an operating pressure of 83 kPa (12 psia). The temperature in the bottom of the column was maintained at about 175°C (347°F). The column had an internal diameter of 15 cm (6 inches), and contained 18 meters (60 feet) of woven wire mesh packing.

The lighter boiling BD and CPD, along with the toluene, was removed as overhead and recycled to the Diels-Alder reactor via a heat exchanger and recycling line. The VNB was removed as a side-stream, along with the VCH and with a small amount of CPD, THI, DCPD, and heavy by-products.

The remaining bottoms contained primarily THI, DCPD, and trimers and other oligimers of CPD, BD, and acyclics, most of which contained fourteen carbon atoms and were comprised of two CPD monomers and one BD monomer. The bottoms were exposed to the foregoing column conditions for 2 hours during which time approximately 90% of the DCPD in the bottoms cracked to monomeric CPD. The resulting CPD was removed as overhead and recycled to produce additional VNB.

The resulting DCPD-depleted heavy product was removed as a bottoms stream.

The presence of fouling in the heat exchanger providing heat for both the distillation and the cracking of DCPD to CPD was determined by calculating the heat transfer coefficient:$\text{Heat transfer coefficient =} \frac{\text{Heat exchanged}}{\text{(exchanger surface area) x (steam temp-process temp)}}$

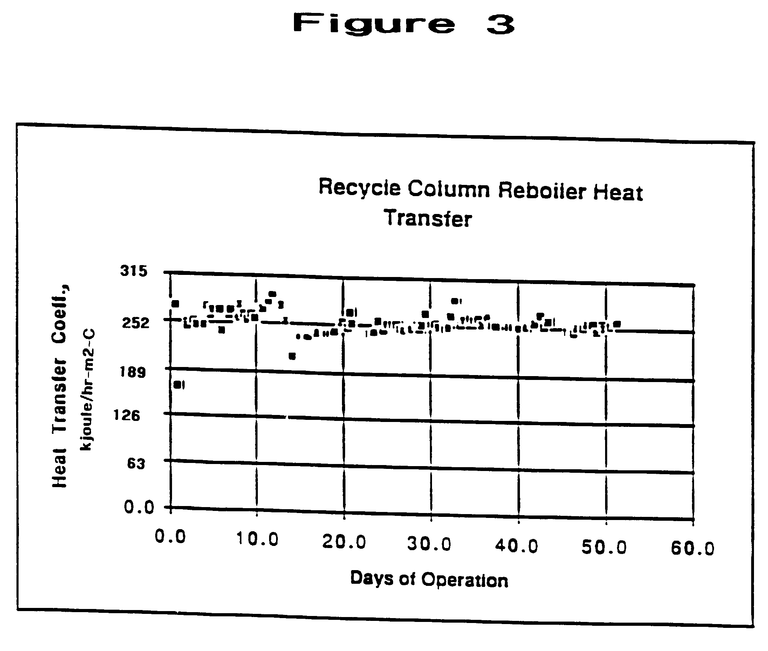

This data is plotted in the **Fig. 3**, and shows no decline in heat transfer coefficient over the duration of the run. This is an indication that fouling is not a problem.

The coefficients are reported in **Fig. 3** at values of kjoule/hr-m²-°C (Btu/hr-ft²-°F), calculated from units of heat exchanged in kjoules/hour (Btu/hour), exchanger surface area in m² (ft²), and temperature in °C (°F). That is

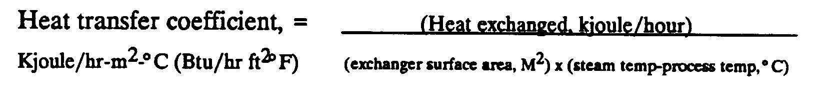

Visual inspection of the reboiler following the total period of the run showed only a very thin film of polymer on the heat transfer surfaces. No polymer collected on the filters in the bottoms product effluent line.

This example illustrates how the invention provides a single, yet economical method, to recover the residual dimer of the cyclic diolefin from the production of an alkenyl bridged ring compound which is accomplished by cracking the dimer to the cyclic diolefin monomer, and recycling the monomer to produce more of the desired alkenyl bridged ring compound without fouling of the equipment.

### EXAMPLE II

The two embodiments of the process of the invention were simulated using a process simulation software package. The flash column was simulated with five stages, and was operated with a reflux ratio of 0.3. Both schemes were run such that the recycle bottoms were treated at the same cracking conditions as in Example I above. Relative mass flows of the CPD monomer and dimer, identified by stream number, are shown in the table below.

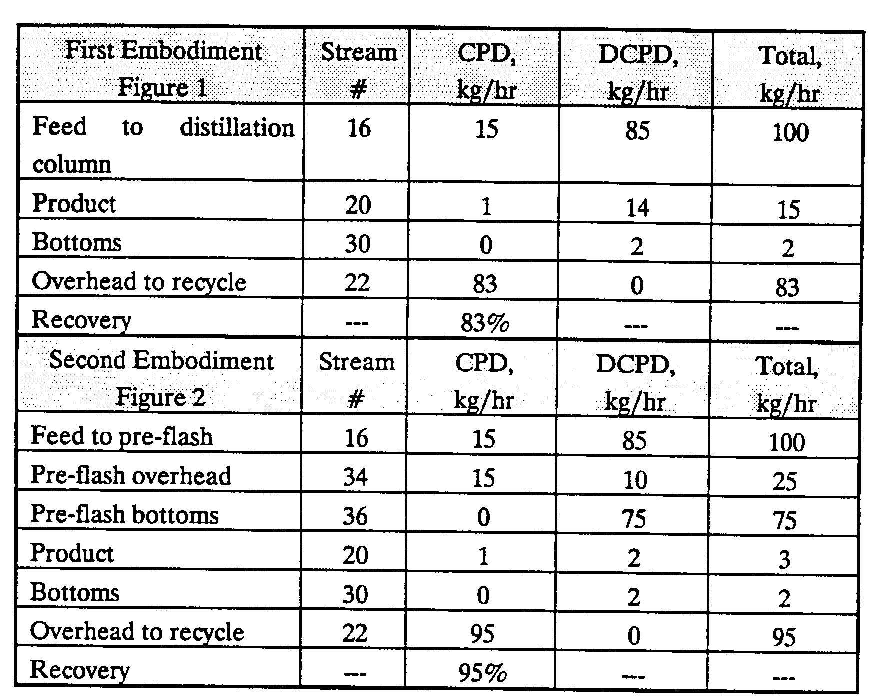

In the first embodiment as illustrated in **Fig. 1**, the overall recovery of CPD + DCPD was 83%. In the second embodiment, with the flash column configuration of **Fig. 2**, the recovery improves to 95%.

This example illustrates the further improvement in recovery using the pre-flash column.

## Claims

1. A process for recovering reactants and products from the reaction of a cyclic diolefin reactant and an olefin reactant to produce an alkenyl bridged ring compound, which process comprises:
(a) introducing into a distillation column at least one effluent feed point, a reaction effluent which comprises unreacted reactant(s), including monomer and dimer forms of the cyclic diolefin, desired alkenyl bridged ring compound and heavy by-products;
(b) removing the alkenyl bridged ring compound from the distillation column as a side stream draw;
(c) removing an overhead from the column comprising the cyclic diolefin, predominantly in monomeric form, leaving a bottoms comprising the cyclic diolefin, predominantly in dimerized form, and heavy byproduct; and
(d) in the column, cracking the dimerized cyclic diolefin contained in the bottoms to recover additional monomeric cyclic diolefin.

2. The process of Claim 1, wherein all or part of the overhead is recycled to the reaction.

3. The process of Claim 1 or 2, wherein the cyclic diolefin reactant comprises monomeric and dimeric cyclic diolefin.

4. The process of any preceding claim, wherein at least some of the make up of cyclic diolefin reactant is provided by feeding dimeric cyclic diolefin to the distillation column, cracking the dimer to monomer in the column bottoms and recovering the make up monomeric cyclic diolefin as column overhead.

5. The process of any preceding claim, wherein the additional or make up monomeric cyclic diolefin is recovered without removing the bottoms from the distillation column.

6. The process of any preceding claim, wherein the additional or make up cyclic diolefin is recovered as distillation column overhead.

7. The process according to any preceding claim, wherein the effluent is introduced into the distillation column without intermediate separation, and the alkenyl bridged ring compound side stream is removed at a location below the effluent feed point.

8. The process according to any one of Claims 1 to 7, which comprises, prior to its introduction into the distillation column, subjecting the effluent to intermediate separation in a flash column to an overhead component (a) depleted in dimeric cyclic diolefin and a bottoms component (b) enriched in dimeric cyclic diolefin; feeding component (a) into the distillation column at an effluent feed point above the side stream draw; and feeding component (b) into the distillation column at an effluent feed point below the side stream draw.

9. The process of any preceding claim, wherein the bottoms is cracked for at least fifteen minutes, preferably at least one hour.

10. The process of any preceding claim, wherein the bottoms is cracked at a pressure of from 3.5-207 kPa (5-30 psia), preferably from between 75.8 - 117.2 kPa (11-17 psia).

11. The process of any preceding claim, wherein the bottoms is cracked at a temperature of about 170-190°C (338-374°F).

12. The process of any preceding claim, wherein the cyclic diolefin comprises 1-methyl-1,3-cyclopentadiene, 2-methyl-1,3-cyclopentadiene, 5-methyl-1,3-cyclopentadiene, 1,3-cyclopentadiene, dicyclopentadiene or mixtures thereof.

13. The process of any preceding claim, wherein the olefin comprises butadiene.

14. The process of Claims 12 and 13, wherein the cyclic diolefin is 1,3-cyclopentadiene, the olefin is butadiene, and the alkenyl bridged ring compound is 5-vinyl-2-norbornene.

## Patentansprüche

1. Verfahren zur Gewinnung von Reaktanten und Produkten aus der Reaktion eines cyclischen Diolefinreaktanten und eines Olefinreaktanten zur Herstellung einer alkenylüberbrückten Ringverbindung, bei dem:
(a) in eine Destillationssäule an mindestens einem Abfuhrmaterialzufuhrpunkt ein Reaktionsabfuhrmaterialmaterial eingeführt wird, das nicht-umgesetzten Reaktanten (nicht-umgesetzte Reaktanten), einschließlich monomere und dimere Formen des cyclischen Diolefins, gewünschte alkenylüberbrückte Ringverbindung und schwere Nebenprodukte umfaßt,
(b) die alkenylüberbrückte Ringverbindung aus der Destillationssäule in Form einer Seitenstromentnahme entnommen wird,
(c) ein Kopfprodukt aus der Säule entnommen wird, das cyclisches Diolefin, überwiegend in monomerer Form, umfaßt, wobei ein Bodensumpf zurückbleibt, der das cyclische Diolefin, überwiegend in dimerisierter Form, und schweres Nebenprodukt umfaßt, und
(d) in der Säule das dimerisierte cyclische Diolefin, das in dem Bodensumpf enthalten ist, gecrackt wird, um zusätzliches monomeres cyclisches Diolefin zu gewinnen.

2. Verfahren nach Anspruch 1, bei dem alles oder ein Teil des Kopfprodukts zu der Reaktion zurückgeführt wird.

3. Verfahren nach Anspruch 1 oder 2, bei dem der cyclische Diolefinreaktant monomeres und dimeres cyclisches Diolefin umfaßt.

4. Verfahren nach einem der vorhergehenden Ansprüche, bei dem mindestens etwas der Ergänzung an cyclischem Diolefinreaktant durch Zuführung von dimerem cyclischen Diolefin zu der Destillationssäule, Crackung des Dimers zu Monomer in dem Säulenbodensumpf und Gewinnung des ergänzten monomeren cyclischen Diolefins als Säulenkopfprodukt bereitgestellt wird.

5. Verfahren nach einem der vorhergehenden Ansprüche, bei dem das zusätzliche oder ergänzte monomere cyclische Diolefin ohne Entfernung des Bodensumpfes aus der Destillationssäule gewonnen wird.

6. Verfahren nach einem der vorhergehenden Ansprüche, bei dem das zusätzliche oder ergänzte cyclische Diolefin als Destillationssäulenkopfprodukt gewonnen wird.

7. Verfahren nach einem der vorhergehenden Ansprüche, bei dem das Abfuhrmaterial in die Destillationssäule ohne eine dazwischenliegende Trennung eingeführt wird und der alkenylüberbrückte Ringverbindungsseitenstrom an einer Stelle unterhalb des Abfurmaterialzufuhrpunktes entnommen wird.

8. Verfahren nach einem der Ansprüche 1 bis 7, bei dem das Abfuhrmaterial vor seiner Einführung in die Destillationssäule einer dazwischenliegenden Trennung in einer Flashsäule iu eine Kopfproduktkomponente (a), die an dimerem cyclischen Diolefin verarmt ist, und einer Sumpfkomponente (b), die an dimerem cyclischen Diolefin angereichert ist, getrennt wird, die Komponente (a) in die Destillationssäule an einem Abfurmaterialzufuhrpunkt oberhalb der Seitenstromentnahme zugeführt wird und die Komponente (b) in die Destillationssäule an einem Abfurmaterialzufuhrpunkt unterhalb der Seitenstromentnahme zugeführt wird.

9. Verfahren nach einem der vorhergehenden Ansprüche, bei dem der Bodensumpf mindestens 15 Minuten lang, vorzugsweise mindestens 1 Stunde lang gecrackt wird.

10. Verfahren nach einem der vorhergehenden Ansprüche, bei dem der Bodensumpf bei einem Druck von 3,5 bis 207 kPa (5 bis 30 psia), vorzugsweise von 75,8 bis 117,2 kPa (11 bis 17 psia) gecrackt wird.

11. Verfahren nach einem der vorhergehenden Ansprüche, bei dem der Bodensumpf bei einer Temperatur von etwa 170 bis 190 °C (338 bis 374 °F) gecrackt wird.

12. Verfahren nach einem der vorhergehenden Ansprüche, bei dem das cyclische Diolefin 1-Methyl-1,3-cyclopentadien, 2-Methyl-1,3-cyclopentadien, 5-Methyl-1,3-cyclopentadien, 1,3-Cyclopentadien, Dicyclopentadien oder Mischungen derselben umfaßt.

13. Verfahren nach einem der vorhergehenden Ansprüche, bei dem das Olefin Butadien umfaßt.

14. Verfahren nach Anspruch 12 und 13, bei dem das cyclische Diolefin 1,3-Cyclopentadien ist, das Olefin Butadien ist und die alkenylüberbrückte Ringverbindung 5-Vinyl-2-norbornen ist.

## Revendications

1. Procédé pour recueillir des corps réactionnels et des produits de la réaction d'un corps réactionnel dioléfinique cyclique et d'un corps réactionnel oléfinique pour produire un composé cyclique à pontage alcényle, procédé qui comprend les étapes consistant :
(a) à introduire dans une colonne de distillation, à au moins un point d'introduction d'effluent, un effluent réactionnel qui comprend un ou plusieurs corps réactionnels n'ayant pas réagi, comprenant la forme monomère et la forme dimère de la dioléfine cyclique, le composé cyclique à pontage alcényle désiré et des sous-produits lourds ;
(b) à évacuer le composé cyclique à pontage alcényle de la colonne de distillation sous forme d'un courant de soutirage latéral ;
(c) à évacuer un courant de tête de la colonne, comprenant la dioléfine cyclique, principalement sous forme monomère, en laissant une queue de colonne de distillation comprenant la dioléfine cyclique, principalement sous forme dimérisée, et un sous-produit lourd ; et
(d) dans la colonne, à effectuer le craquage de la dioléfine cyclique dimérisée présente dans la queue de colonne de distillation pour recueillir une quantité supplémentaire de dioléfine cyclique monomère.

2. Procédé suivant la revendication 1, dans lequel la totalité ou une partie du courant de tête est recyclée à la réaction.

3. Procédé suivant la revendication 1 ou 2, dans lequel le corps réactionnel dioléfinique cyclique comprend une dioléfine cyclique sous forme monomère et sous forme dimère.

4. Procédé suivant l'une quelconque des revendications précédentes, dans lequel au moins une partie de la quantité d'appoint du corps réactionnel dioléfinique cyclique est fournie en introduisant la dioléfine cyclique dimère dans la colonne de distillation, en provoquant le craquage de la forme dimère en la forme monomère dans la queue de colonne et en recueillant la dioléfine cyclique monomère d'appoint comme courant de tête de colonne.

5. Procédé suivant l'une quelconque des revendications précédentes, dans lequel la quantité supplémentaire ou d'appoint de dioléfine cyclique monomère est recueillie sans évacuer la queue de colonne de la colonne de distillation.

6. Procédé suivant l'une quelconque des revendications précédentes, dans lequel la quantité supplémentaire ou d'appoint de dioléfine cyclique est recueillie sous forme d'un courant de tête de colonne de distillation.

7. Procédé suivant l'une quelconque des revendications précédentes, dans lequel l'effluent est introduit dans la colonne de distillation sans une séparation intermédiaire, et le courant latéral de composé cyclique à pontage alcényle est évacué à un emplacement situé au-dessous du point d'introduction d'effluent.

8. Procédé suivant l'une quelconque des revendications 1 à 7, qui comprend, avant son introduction dans la colonne de distillation, l'étape consistant à soumettre l'effluent à une séparation intermédiaire dans une colonne de détente donnant un constituant de tête (a) appauvri en dioléfine cyclique dimère et un constituant de queue de colonne (b) enrichi en dioléfine cyclique dimère ; à introduire le constituant (a) dans la colonne de distillation à un point d'introduction d'effluent situé au-dessus du courant de soutirage latéral ; et à introduire le constituant (b) dans la colonne de distillation à un point d'introduction d'effluent situé au-dessous du courant de soutirage latéral.

9. Procédé suivant l'une quelconque des revendications précédentes, dans lequel la queue de colonne est soumise à un craquage pendant au moins quinze minutes, de préférence pendant au moins une heure.

10. Procédé suivant l'une quelconque des revendications précédentes, dans lequel la queue de colonne est soumise à un craquage à une pression de 3,5 à 297 kPa (5 à 30 psia), de préférence de 75,8 à 117,2 kPa (11 à 17 psia).

11. Procédé suivant l'une quelconque des revendications précédentes, dans lequel la queue de colonne est soumise à un craquage à une température d'environ 170 à 190°C (338 à 374°F).

12. Procédé suivant l'une quelconque des revendications précédentes, dans lequel la dioléfine cyclique comprend le 1-méthyl-1,3-cyclopentadiène, le 2-méthyl-1,3-cyclopentadiène, le 5-méthyl-1,3-cyclopentadiène, le 1,3-cyclopentadiène, le dicyclopentadiène ou leurs mélanges.

13. Procédé suivant l'une quelconque des revendications précédentes, dans lequel l'oléfine comprend le butadiène.

14. Procédé suivant les revendications 12 et 13, dans lequel la dioléfine cyclique consiste en 1,3-cyclopentadiène, l'oléfine consiste en butadiène et le composé cyclique à pontage alcényle consiste en 5-vinyl-2-norbornène.
